Europäisches Patentamt

European Patent Office

Office européen des brevets

(19)

(11) Publication number: **0 235 457**
A2

(12) # EUROPEAN PATENT APPLICATION

(21) Application number: **86309731.7**

(22) Date of filing: **12.12.86**

(51) Int. Cl.⁴: **C 12 P 21/00, C 07 K 15/00, C 12 N 5/00, C 12 N 15/00, G 01 N 33/577, B 01 D 43/00 // (C12P21/00, C12R1:91)**

(30) Priority: **13.12.85 GB 8530829**

(71) Applicant: **SYNGENE INTERNATIONAL N.V., Plaza Jojo Correa 1-5, Willemstad Curaçao Netherlands Antilles (NL)**

(43) Date of publication of application: **09.09.87**
Bulletin 87/37

(72) Inventor: **Finnegan, Irene, 3 Garthorpe Road, Northern Moor Manchester 23 (GB)**
Inventor: **Heath, John Francis, Grey Tiles Mid Lavant, Midchester West Sussex (GB)**
Inventor: **Dolamore, Peter William, Grey Tiles Mid Lavant, Midchester West Sussex (GB)**

(84) Designated Contracting States: **AT BE CH DE ES FR GB GR IT LI LU NL SE**

(74) Representative: **Froud, Clive et al, Elkington and Fife High Holborn House 52/54 High Holborn, London WC1V 6SH (GB)**

(54) **Monoclonal antibodies.**

(57) A monoclonal antibody to a metal salt or a metal complex is produced by a hybridoma cell line formed by in vitro fusion of mouse spleen cells which have been sensitized to the metal salt or the metal complex and cells from a myeloma cell line, e.g. P3-AgX-653. The monoclonal antibody may be used in a process for the recovery of metal values from a solution or a dispersion or a slurry or the like containing a metal salt or a metal complex which includes the steps of contacting the solution or the like containing the metal salt or the metal complex with a support on which are immobilized monoclonal antibodies to the metal salt or the metal complex so that a complex is formed between the monoclonal antibodies and the metal salt or the metal complex, and when the support has reached the desired metal value loading, eluting the metal values from the support with a suitable eluant and collecting the eluate containing the metal values. The monoclonal antibody may also be used in a method of detecting the presence of a metal salt or a metal complex in a sample.

-1-

"MONOCLONAL ANTIBODIES"

## BACKGROUND OF THE INVENTION

This invention relates to monoclonal antibodies to organic and inorganic metal salts and metal complexes, to hybrid cell lines (hybridomas) which may be used in the preparation of such monoclonal antibodies, and to the use of the monoclonal antibodies in the detection of metal salts and metal complexes, and in the recovery of metal values from solutions or slurries or the like containing such values.

The fusion of mouse myeloma cells to mouse spleen cells (splenocytes) sensitized to a particular antigen in order to produce hybridomas which secrete monoclonal antibodies is a known art (Koehler and Milstein, Nature, 256, 495 (1975), and improvements by Galfre, Howe, Milstein, Butcher and Howard, Nature 266, 550 (1977); Galfre, Milstein and Wright, Nature, 277, 131 (1979); and Shulman and Koehler, Nature, 276, 269 (1978)). However there are many difficulties to be overcome and variations required for each specific case.

## SUMMARY OF THE INVENTION

According to a first aspect of the invention there is provided a monoclonal antibody to a metal salt or a metal complex, the monoclonal antibody being produced by a hybridoma cell line formed by fusion of animal spleen cells which have been sensitized to the metal salt or the metal complex and cells from a myeloma cell line.

The sensitization may be carried out in vivo or preferably in vitro.

The animal spleen cells and the myeloma cell line are preferably mouse spleen cells and a mouse myeloma cell line. Specifically, the monoclonal antibodies are preferably produced by a hybridoma formed by the fusion of sensitized mouse spleen cells with the mouse myeloma cell line P3-AgX-653.

The monoclonal antibody may be a monoclonal antibody to an organic or an inorganic metal salt or metal complex. Preferably, the monoclonal antibody is a monoclonal antibody to a metal salt selected from gold cyanide, silver cyanide, platinum (IV) chloride, rhodium (III) chloride, iridium (IV) chloride, rhodium (III) bromide, platinum (II) chloride, palladium (II) chloride, palladium (II) bromide and iridium (III) chloride. Most preferably, the monoclonal antibody is a monoclonal antibody to gold cyanide.

The monoclonal antibody is preferably of the class IgG.

According to a second aspect of the invention there is provided a hybridoma formed by fusion of animal spleen cells previously sensitized to a metal salt or a metal complex and cells from a myeloma cell line, the hybridoma being capable of producing a monoclonal antibody as described above.

According to a third aspect of the invention there is provided a method of producing a hybridoma as described above including the steps of:

0235457

(i) sensitizing animal spleen cells to a metal salt or a metal complex;

(ii) fusing the sensitized spleen cells with myeloma cells in the presence of a cell fusion promoter; and

(iii) selecting and cloning hybridomas which produce the desired monoclonal antibody.

According to a fourth aspect of the invention there is provided a process for the recovery of metal values from a solution or a dispersion or a slurry or the like containing a metal salt or a metal complex including the steps of:

(i) contacting the solution or the dispersion or the slurry or the like containing the metal salt or the metal complex with a support on which are immobilized monoclonal antibodies to the metal salt or the metal complex so that a complex is formed between the monoclonal antibodies and the metal salt or the metal complex; and

(ii) when the support has reached the desired metal value loading, eluting the metal values from the support with a suitable eluant and collecting the eluate containing the metal values.

According to a fifth aspect of the invention there is provided a monoclonal antibody as described above for use in an immunoassay method of detecting the presence of a metal salt or a metal complex in a sample.

BRIEF DESCRIPTION OF THE DRAWINGS

Figure 1 is a graph of the results of a direct ELISA for an anti-gold cyanide antibody hybridoma cell line designated AUCN-8511 produced as described hereinafter; and

Figures 2 and 3 are graphs of the results of two 50% displacement assays for the anti-gold cyanide antibody hybridoma cell line designated AUCN-8511.

## DESCRIPTION OF PREFERRED EMBODIMENTS

An example of the invention, viz. the production of a hybridoma which produces monoclonal antibodies to gold cyanide will now be described.

The hybridoma is produced from mouse splenocytes sensitized to gold cyanide fused with mouse myeloma cells from the cell line P3-AgX-653.

The process comprises the following steps:

1. $25cm^3$ tissue culture flasks with angle necks (Nunc) are coated with 5ml of a gold cyanide (EGA-Chemie cat. no. 16.118-7) suspension of 10µg/ml in a carbonate coating buffer (pH 9,6), consisting of 1,6g/l $Na_2CO_3$ and 3,0g/l $NaHCO_3$ in distilled water. The gold cyanide standard is weighed and autoclaved prior to use and added under sterile conditions to filter-sterilised carbonate coating buffer. This sterile preparation is sonicated for three hours to facilitate the uptake of gold cyanide into solution as a finely divided suspension. 5ml aliquots are dispersed using aseptic techniques, into the tissue culture flasks.

2. The flasks are incubated for one hour at 37°C or overnight at 4°C and then washed out with three aliquots of 5ml of Dulbecco's Modified Eagles Medium (DMEM - Gibco cat. no. 079-3016) containing 25mM HEPES buffer and sodium bicarbonate at the reduced level of 0,85g/l. This basal media is obtained in powdered form and made up and sterile filtered using Millipore filtration equipment.

3. Female Balb/C mice (six to ten weeks old) are killed by cervical dislocation and dipped in 70% ethanol. The spleen is removed aseptically and placed in a petri dish containing DMEM. The spleen is disaggregated using two bent hypodermic needles and the cells broken up into a single cell suspension with gentle pipetting using a wide nose serological pipette. The cells are washed three times by centrifugation (10 times at 1000 r.p.m. at ambient temperature) in DMEM in round bottom screw cap tubes (tissue culture quality - Nunc). These cells are resuspended on the final wash in complete DMEM (DMEM containing 10% fetal calf serum - Gibco cat. no. 011-6290, previously heat inactivated at 56°C for 30 minutes and 2% of 200mM Glutamine - Gibco cat. no. 043-5030) to a cell density of $5x10^6$/ml. 5ml of this is placed in each coated flask.

4. The flasks are incubated for 3-4 days at 37°C or until blast formation is observed. This can be seen as clones of cells growing in clumps on the floor of the tissue culture flask with (usually) a monocyte as the focus of their growth. These clones eventually lift off the bottom of the flask and form cones of cells growing in suspension (they can reach quite large dimensions and are just visible with the naked eye).

5. The sensitized splenocytes are removed from the flask by vigorous shaking and by scraping the bottom of the flask with a sterile, angled scraper (Flow Laboratories).

6. The cells are washed three times by centrifugation in DMEM at 1000 x g for 10 minutes and at ambient temperature. At the same time cells harvested from cultures of P3-AgX-653 (in the log phase of growth and also grown in complete DMEM, as described above) are washed in the same way.

7. The sensitized splenocytes and myeloma cells are then counted and combined in a ratio of 10:1 viable cells in a round bottomed tissue culture grade centrifuge tube and co-centrifuged to form a layer of cells on the bottom of the tube.

8. The supernatant is gently aspirated off, leaving approximately 100μl of residual DMEM to cover the cells.

9. The pelleted cells are then gently tapped to remove them from the bottom of the tube.

10. Fusing mixture at a volume of 100µl is added dropwise to the tube containing the combined splenocytes and myeloma cells, from a pasteur pipette, over a period of two minutes.

Fusing mixture (30% polyethylene glycol 1500 - BDH; 5% dimethylsulphoxide - Sigma cat. no. D5879; in DMEM) is prepared as follows:

Polyethylene glycol (PEG) is autoclaved a minimum of three times and then cooled to a temperature at which it remains liquid. Under aseptic conditions 3ml of PEG is added quickly to prewarmed DMEM (approximately 5ml) and 0,5ml DMSO is added to this. The pH is adjusted to be slightly alkaline (using the color of the phenol red indicator in the DMEM) using sterile 1N NaOH and the solution made up to 10ml with DMEM.

11. The mixture is then diluted to 10ml with basal DMEM in the following way:

0,5ml added dropwise over 2 minutes
1,0ml added dropwise over 2 minutes
2,0ml added dropwise over 2 minutes
2,0ml added dropwise over 2 minutes
2,0ml added dropwise over 2 minutes
2,5ml added dropwise over 2 minutes

This is judged roughly with a stopwatch and is carried out at ambient temperature. The cell suspension is centrifuged at 400 x g for 10 minutes (ambient temperature), during which the cells "tag" together with the PEG used. DMSO enhances the conditions under which fusion takes place.

12. The supernatant is aspirated off and the cells are gently resuspended in 5ml of complete basal media and the whole volume placed in a 25cm$^3$ tissue culture flask.

13. Twenty four hours later an equal volume of HAT (hypoxanthine-aminopterin-thymidine, obtained as a 50x concentrate from Gibco-cat.no. 043-1060) in complete DMEM, is added. This selects for hybrid cells as the unfused parent myeloma cells are killed by aminopterin, due to their inability to salvage hypoxanthine and thymidine from the exogenous media by the alternative purine salvage pathway. Unfused parent splenocytes usually die within 4-7 days but their continued presence has been noted over longer periods in some cases.

14. The primary culture is incubated at 37°C until good hybrid growth is observed. This is variable and appears to depend on the donor mouse splenocytes. The cells are fed twice a week by partial removal of tissue culture supernatant and replacement with fresh complete media. Hereafter the cells are maintained at $10^6$ cells/ml.

15. Tissue culture supernatant is removed for testing by ELISA (enzyme-linked-immunosorbant-assay). Supernatant is removed and clarified by centrifugation for 15 minutes at 5000 x g and stored at 4°C prior to use.

Testing is done in one of three ways:

a. Direct EIA (enzyme immunoassay)
b. Sandwich EIA
c. 50% Displacement Assay.

a. Direct EIA

Gold cyanide suspension made up as detailed in step 1 above is diluted to the desired concentration in carbonate coating buffer (0-100µg/ml) and 100µl is dispensed per well of a 96-well EIA plate (Nunc). These plates are incubated at 37°C for 2 hours or overnight at 4°C. The plates are washed three times in tris-saline washing buffer (0,02M tris-HCl and 0,2M NaCl - pH 7,4). The plates are then "blocked" to take up unoccupied binding sites on the plate, using a 2mg/ml bovine serum albumin solution (Sigma cat.no. A7638) in tris-saline buffer (0,02M tris-HCl and 0,2M NaCl - pH 9,6) and are then washed three times in Tris-saline washing buffer.

An amount of 100µl of the antibody supernatant is added per well and then incubated for one hour at 37°C and washed in tris-saline buffer pH 7,4, containing Tween 20 (0,05% v/v - Sigma cat.no. P1379) three times and once in distilled water.

50µl of an anti-mouse IgG conjugate (sheep F(ab')$_2$ anti-mouse IgG alkaline phosphatase conjugate, diluted according to the manufacturer's instructions (eg. Biorad Clone Selector Kit) is added to each well and the plates are incubated at 37°C for one hour. The plates are washed three times in tris-saline-Tween washing buffer and once in distilled water. 50µl of substrate (p-nitrophenolphosphate, 0,05M in distilled water diluted 1/10, 10mM 2-amino-2-methyl-1-propanol (Merck) and 1mM MgCl$_2$ pH 10,2) is added to each well. The plates are developed at 37°C and the reaction stopped with 0,5M NaOH. The absorbance of each well is read relative to a reagent blank at 410nm using a Biorad model 2550 EIA spectrophotometer. The plates include a control of an unrelated mouse-hybridoma supernatant, in order to check for non-specific binding.

b.  Sandwich EIA

Tissue culture supernatant is concentrated ten times using a Millipore tangential flow filtration bed (Millitan TM) through a 10kd molecular cut-off filter. This solution is diluted 1/10 in carbonate coating buffer and coated onto 96-well EIA plates at 100µl per well. The ELISA procedure is then carried out exactly as detailed above (a. Direct EIA), excepting that dilutions of gold cyanide were made up in tris-saline buffer pH 7,4. This was found to improve the reproducability of binding of antigen to the EIA plates.

c. 50% Displacement Assay

The techniques in a. and b. above are used to optimize the concentration of antigen binding. The dislacement assay described here is used to check the percentage of cross-reactivity with other metal salts and complexes, in the following way:

(i) The EIA plate is coated with the optimal concentration of antigen as detailed in a. and b. above. It is then incubated, washed, blocked and washed again as in a.

(ii) 50µl of cell line supernatant and 50µl of competing antigen solution is added per well. Competing antigen is made up in tris-saline solution pH 7,4. The plates are then incubated for two hours and the ELISA procedure continued as in a. The concentration of competing antigen causing 50% displacement of target antigen can then be assessed by plotting $\Delta$OD against competing antigen concentration. (This technique is detailed in the literature - Candlish, Stimson and Smith, Letters in Applied Microbiology 1, 57-61, 1985).

16. Cell lines showing good antibody production are put into bulk culture in "Cell Factories" (Nunc), at a concentration of $10^6$ cells/ml and maintained by partial replacement of cell line supernatants. The "factory" is maintained at 37°C although certain cell lines produce more antibody at slightly lower temperatures.

17. Pooled, clarified cell line supernatants are stored at 4°C and concentrated ten times using a Millipore Millitan (TM) system with a 10kd cut-off filter. Concentrated supernatants are further concentrated by a 50% ammonium sulphate (Merck) precipitation, followed by extensive dialysis against phosphate buffered saline. Partially purified antibody is isolated on a Protein A affinity column (Biorad - Affigel Protein A Affinity Column Kit). Purified antibody product is either stored at 4°C or aliquoted and frozen.

Antibody is isotyped using a Biorad isotyping kit or by using an Ouchterlony procedure. Due to the way that the hybridoma cell lines are produced, all IgG isotypes appear in the tissue culture supernatants; IgM appears variably and IgA is absent.

An anti-gold cyanide antibody hybridoma cell line designated AUCN-8511 produced by following the procedure described above was subjected to various tests and the results thereof are given below.

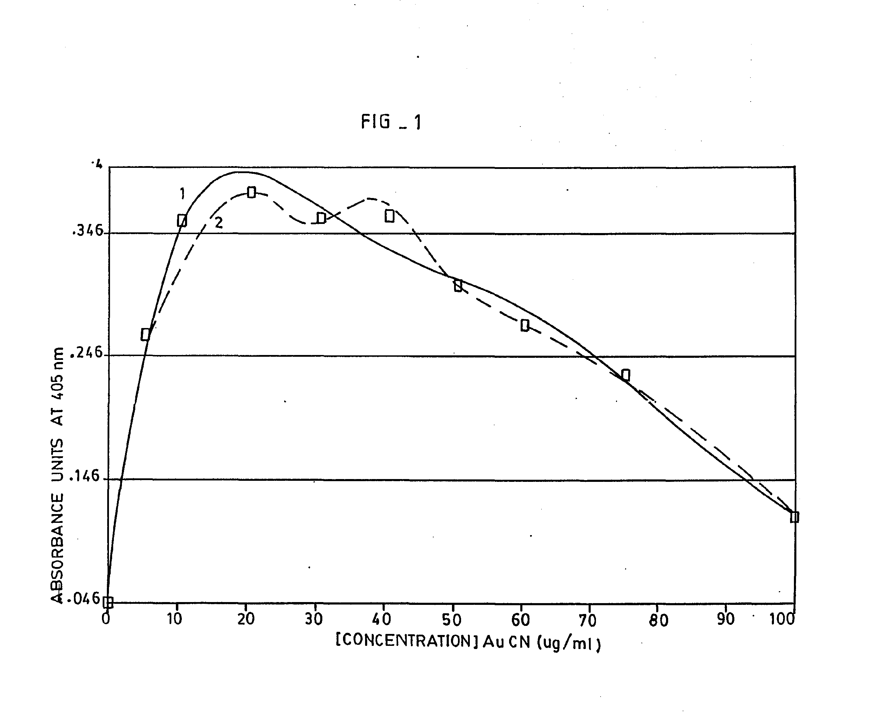

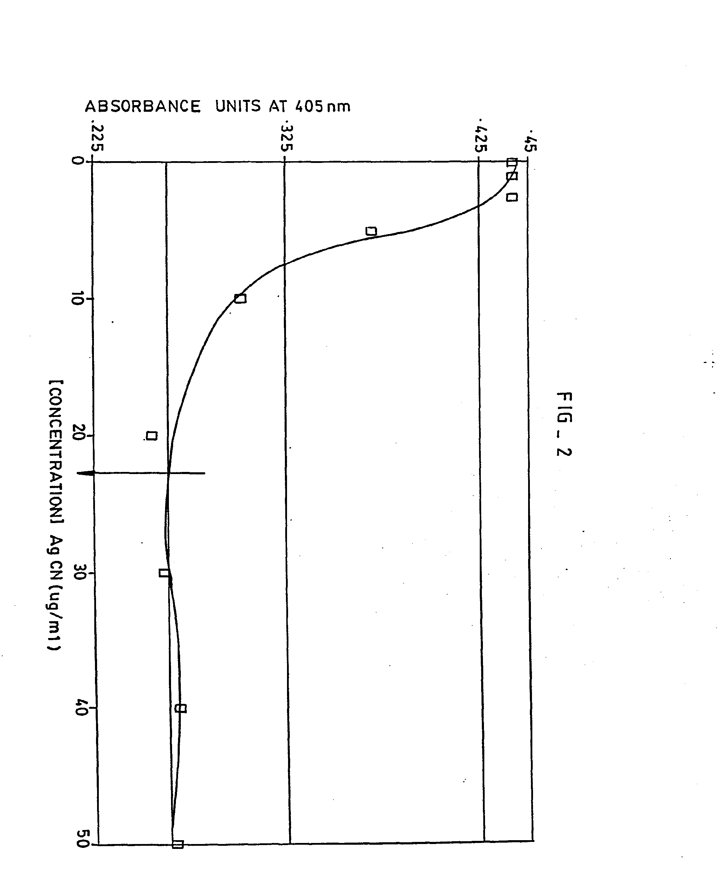

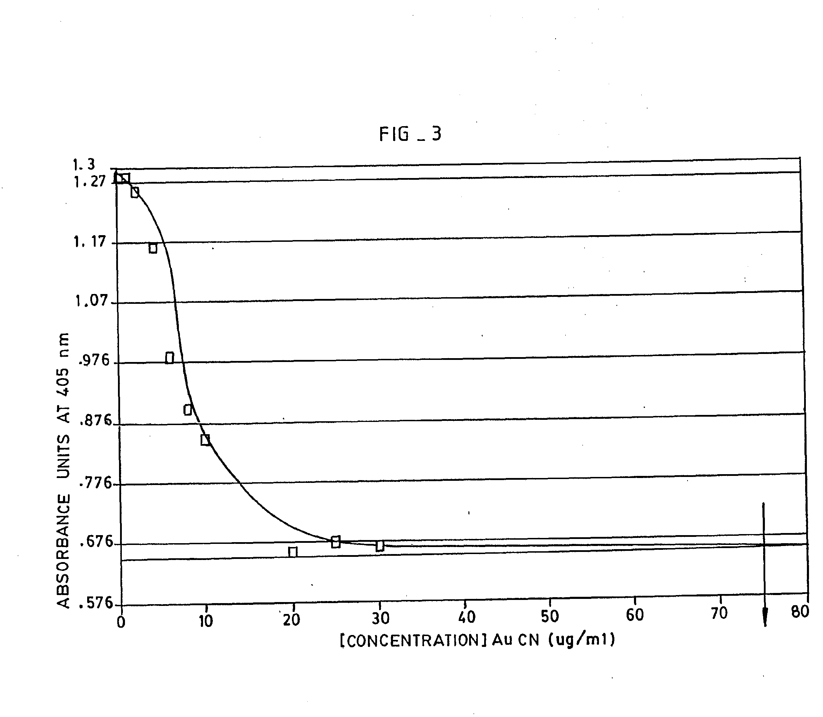

The results of a direct ELISA are presented graphically in Figure 1.
Additionally, the results of two 50% displacement assays are presented in Figures 2 and 3 respectively.

Figure 2 shows the displacement of anti-gold cyanide antibody with silver cyanide. The concentration of silver cyanide (Aldrich Catalogue No. 18,453-5) giving 50% maximum absorbance is 7µg/well which is equivalent to 70µg/ml.

Figure 3 shows the displacement of anti-gold cyanide antibody with gold cyanide. The concentration of gold cyanide giving 50% maximum absorbance is 2,25µg/well which is 22,5µg/ml.

The cross-reactivity of the anti-gold cyanide antibody produced by cell line AUCN-8511 is therefore:

22,5/76 X 100% = 29,6%.

In a similar manner, monoclonal antibodies to the following metal salts or complexes were made:

Silver cyanide
Platinum (IV) chloride
Rhodium (III) chloride (Strem Chemicals cat.no. 45-1878)
Eridium (IV) chloride (Strem Chemicals cat.no. 93-7727)
Rhodium (III) bromide (Strem Chemicals cat.no. 45-1876)
Platinum (II) chloride (Strem Chemicals cat.no. 78-1480)
Palladium (II) chloride (Strem Chemicals cat.no. 46-1850)
Palladium (II) bromide (Strem Chemicals cat.no. 46-1836)
Iridium (II) chloride (Strem Chemicals cat.no. 93-7729)

Monoclonal antibodies may also be made to other salts and complexes of gold, silver, rhodium, iridium, platinum and palladium, as well as to salts and complexes of other metals such as the rare earth metals.

For use in the process of the invention, the monoclonal antibodies are immobilised on a suitable support or a carrier such as glass beads, a microporous plastic sheet, oxirane acrylic beads or the like. The immobilisation of proteins like monoclonal antibodies on a microporous plastic sheet is detailed in US Patent No. 4,169,014 (Amerace Corporation). Further, monoclonal antibodies of the IgG class can be covalently immobilised onto styrene, nylon and acrylic acid using different chemical techniques (see McManus and Randerson, Chem. Eng. J., 32 (1986) B19-B28).

An example of the use of monoclonal antibodies to gold cyanide, immobilised on acrylic beads is given below.

The acrylic beads are placed in a suitable column or the like. Thereafter a solution or a pulp or a slurry or the like containing gold cyanide values is passed through the column.

The gold cyanide forms a complex with the monoclonal antibodies and is thus removed from the solution or the pulp or the slurry or the like and remains on the support. When the support is substantially fully loaded, the gold values may be eluted from the support by passing a common eluent or release agent such as a solution having a different pH or ionic strength through the column to elute the gold values. The eluent may be then collected and the gold values recovered in the usual way. The column may then be reused for the extraction of further gold values.

The monoclonal antibodies to metal salts or complexes may be used to extract metal values from slurries or solutions obtained from mining processes or from solutions containing very low concentrations of such values such as seawater or spent plating solutions or the like.

Further, the monoclonal antibodies of the invention may be used in a method of detecting the presence of a metal salt or a metal complex in a sample using a modification of conventional immunassay procedures. This could be, for example a modification of the Sandwich ELISA technique. In terms of this method, a monoclonal antibody to a specific metal salt or metal complex is immobilised on a support. Thereafter, the sample which may or may not contain the metal salt or the metal complex is brought into contact with the monoclonal antibody to allow any metal salt or metal complex present to be bound by the monoclonal antibody. Thereafter, a metal-antibody-enzyme conjugate is added so that a complex is formed which gives a visual colour change varying in intensity according to the amount of antigen present.

Thus the monoclonal antibodies of the invention may also form part of a diagnostic kit for the detection of a particular metal salt or metal complex.

Another use of the monoclonal antibodies of the invention lies in the development of biosensors, similar to an application which has been developed using Eupergit (TM)(See Wehnert et al, Biotechnology letters, Vol.7, No.11,837-840)(1985); Huck et al, Analyst, Vol.109, Feb.1984.)

CLAIMS:

1.
A monoclonal antibody to a metal salt or a metal complex, the monoclonal antibody being produced by a hybridoma cell line formed by fusion of animal spleen cells which have been sensitized to the metal salt or the metal complex and cells from a myeloma cell line.

2.
A monoclonal antibody according to claim 1 wherein the animal spleen cells are mouse spleen cells and wherein the myeloma cell line is a mouse myeloma cell line.

3.
A monoclonal antibody according to claim 2 wherein the mouse myeloma cell line is mouse myeloma cell line P3-AgX-653.

4.
A monoclonal antibody according to any one of claims 1 to 3 of the type IgG.

5.
A monoclonal antibody according to any one of claims 1 to 4 wherein the metal salt or metal complex is an organic metal salt or an organic metal complex.

6.
A monoclonal antibody according to any one of claims 1 to 4 wherein the metal salt or metal complex is an inorganic metal salt or an inorganic metal complex.

7.
A monoclonal antibody according to any one of claims 1 to 4 wherein the monoclonal antibody is a monoclonal antibody to a metal salt or a metal complex selected from gold cyanide, silver cyanide, platinum (IV) chloride, rhodium (III) chloride, iridium (IV) chloride,
rhodium (III) bromide, platinum (II) chloride, paladium (II)
chloride, paladium (II) bromide, and iridium (III) chloride.

8.

A monoclonal antibody according to claim 7 wherein the monoclonal
antibody is a monoclonal antibody to gold cyanide.

9.

A hybridoma formed by fusion of animal spleen cells previously
sensitized to a metal salt or a metal complex and cells from a
myeloma cell line, the hybridoma being capable of producing a
monoclonal antibody to the metal salt or the metal complex.

10.

A hybridoma according to claim 9 formed by fusion of mouse spleen
cells and cells from a mouse myeloma cell line.

11.

A hybridoma according to claim 10 wherein the mouse myeloma cell
line is mouse myeloma cell line P3-AgX-653.

12.

A hybridoma according to any one of claims 9 to 11 capable of
producing a monoclonal antibody of the type IgG.

13.

A hybridoma according to any one of claims 9 to 12 capable of
producing a monoclonal antibody to a metal salt selected from gold
cyanide, silver cyanide, platinum (IV) chloride, rhodium (III)
chloride, iridium (IV) chloride, rhodium (III) bromide, platinum
(II) chloride, paladium (II) chloride, paladium (II) bromide, and
iridium (III) chloride.

14.

A method of producing a hybridoma according to any one of claims 9
to 13 including the steps of:

(i) sensitizing animal spleen cells to a metal salt or a metal complex;

(ii) fusing the sensitized spleen cells with myeloma cells in the presence of a cell fusion promoter; and

(iii) selecting and cloning hybridomas which produce the desired antibody.

15.

A process for the recovery of metal values from a solution or a dispersion or a slurry containing a metal salt or a metal complex including the steps of:

(i) contacting the solution or the dispersion or the slurry containing the metal salt or the metal complex with a support on which are immobilised monoclonal antibodies to the metal salt or the metal complex so that a complex is formed between the monoclonal antibodies and the metal salt or the metal complex; and

(ii) when the support has reached the desired metal value loading, eluting the metal values from the support with a suitable eluant and collecting the eluate.

16.

A process according to claim 15 wherein the solution or the dispersion or the slurry contains gold cyanide.

17.

A monoclonal antibody according to any one of claims 1 to 8 for use in an immunoassay method of detecting the presence of a metal salt or a metal complex in a sample.

FIG _ 1

FIG - 2

FIG _ 3